**Europäisches Patentamt**

**European Patent Office**

**Office européen des brevets**

(11) Veröffentlichungsnummer: **0 062 002
B1**

(12) # EUROPÄISCHE PATENTSCHRIFT

(45) Veröffentlichungstag der Patentschrift:
**04.06.86**

(21) Anmeldenummer: **82810129.5**

(22) Anmeldetag: **19.03.82**

(51) Int. Cl.⁴: **A 61 K 7/11**

(54) **Haarfixierungszubereitung, deren Herstellung und deren Verwendung in Aerosolsprays.**

(30) Priorität: **25.03.81 CH 2019/81**

(43) Veröffentlichungstag der Anmeldung:
**06.10.82 Patentblatt 82/40**

(45) Bekanntmachung des Hinweises auf die Patenterteilung:
**04.06.86 Patentblatt 86/23**

(84) Benannte Vertragsstaaten:
**CH DE FR GB IT LI**

(56) Entgegenhaltungen:
**EP - A - 0 037 378
FR - A - 2 424 738
FR - A - 2 450 861
US - A - 3 025 219
US - A - 3 577 517
US - A - 3 927 199**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel (CH)**

(72) Erfinder: **Hungerbühler, Walter, Höhenstrasse 8, CH-4125 Riehen (CH)**
Erfinder: **Meindl, Hubert, Dr., Kornfeldstrasse 77, CH-4125 Riehen (CH)**

ACTORUM AG

**Beschreibung**

Vorliegende Erfindung betrifft eine Haarfixierungszubereitung mit einem Terpolymeren auf Acrylbasis als Hauptwirksubstanz in Form eines wasserfreien oder wasserhaltigen Aerosolsprays unter Verwendung von nicht-halogenierten Treibgasen.

In letzter Zeit werden aus ökologischen Gründen halogenhaltige Treibgase immer mehr durch halogenfreie ersetzt. Es wurde jedoch festgestellt, dass carboxylierte Harze in alkoholischen Formulierungen, wie sie vorzugsweise als Aerosol-Haarsprays verwendet werden, in Gegenwart halogenfreier Treibgase, wie z.B. Propan, Butan, Isobutan oder deren Gemische, eine geringere Löslichkeit aufweisen als bei Verwendung von Fluorchlorkohlenwasserstoffen. Dies kann zu Ausfällungen der Polymeren während der Lagerzeit führen, wodurch die Sprays unbrauchbar werden.

In der DE-A-2 917 504 werden beispielsweise langkettige Amine als Neutralisationsmittel vorgeschlagen, welche die Verträglichkeit der Polymere mit den genannten Treibgasen erhöhen. In der DE-A-2 317 484 oder US-A-3 927 199 können bei Verwendung von Polymeren, die als einen der Bausteine t-Octylacrylamid enthalten, halogenierte Treibgase mit geringen Anteilen Isobutan eingesetzt werden. Dabei bewirkt der Einsatz des langkettigen t-Octylacrylamides eine bessere Treibgasverträglichkeit als kürzerkettige Acrylamide.

Es wurde nun gefunden, dass Terpolymere, welche aus Monomeren mit Substituenten von höchstens 4 Kohlenstoffatomen erhalten werden und deren Carboxylgruppen durch die in der Kosmetik gebräuchlichen kurzkettigen Aminoalkohole neutralisiert werden, eine so gute Verträglichkeit mit halogenfreien Treibgasen aufweisen, dass diese in Aerosolsprayformulierungen 30–100% der Freon-Treibgase ersetzen können.

Gegenstand vorliegender Erfindung ist demnach eine Haarfixierungszubereitung, welche dadurch gekennzeichnet ist, dass sie

(1) eine Lösung von mindestens einem Terpolymeren in einem wasserfreien oder wasserhaltigen organischen Lösungsmittel, wobei das Terpolymer durch Copolymerisation

(a) von 40 bis 60 Gew.-% eines im Alkylteil 1 bis 4 Kohlenstoffatome aufweisenden N-Alkylacrylamides oder N-Alkylmethacrylamides mit

(b) 35 bis 50 Gew.-% eines $C_1$–$C_4$-Hydroxyalkylesters oder vorzugsweise $C_1$–$C_4$-Alkylesters der Acrylsäure oder Methacrylsäure und

(c) 3 bis 11 Gew.-% Acrylsäure oder Methacrylsäure, bezogen auf das Gesamtgewicht des Monomerengemisches, hergestellt und mindestens 50% der im Terpolymer vorhandenen Carboxylgruppen mit einer niederen organischen Base ausgewählt aus Triethanolamin, Triisopropanolamin, 2-Amino-2-methyl-propanol und 2-Amino-2-methyl-1,3-propandiol neutralisiert worden ist, und

(2) ein Treibmittel, welches aus mindestens 30 Gew.-% bezogen auf das gesamte Treibmittel, eines halogenfreien Treibgases besteht, enthält.

Die als Monomere (a) verwendbaren N-substituierten Acrylamide oder Methacrylamide sind mit Alkylresten substituiert, welche 1 bis 4 Kohlenstoffatome enthalten. Beispiele für derartige anwendbare Acrylamide und Methacrylamide sind N-Methylacrylamid, N-Ethylacrylamid, N-Propylacrylamid, N-n-Butylacrylamid, N-Isopropylacrylamid, N-sek-Butylacrylamid, N-tert.-Butylacrylamid sowie die entsprechenden Methacrylamide. Bevorzugt sind die Acrylamide und insbesondere N-tert.-Butylacrylamid.

Beispiele von als Monomeren (b) verwendbaren Alkylester der Acrylsäure oder Methacrylsäure sind Methyl-, Ethyl-, Propyl-, Isopropyl- oder n-Butylacrylat oder -methacrylat, wobei die Acrylate gegenüber den Methacrylaten bevorzugt sind. Dabei ist Acrylsäureethylester besonders bevorzugt. Des weiteren können Acrylate oder Methacrylate mit Hydroxylgruppen, wie z.B. Hydroxyethylacrylat, Hydroxypropylacrylat oder Hydroxyethylmethacrylat als Komponente (b) verwendet werden.

Besonders bevorzugte Komponente (c) ist Acrylsäure.

Die Copolymerisation zur Herstellung der erfindungsgemäss verwendbaren Terpolymeren wird vorteilhafterweise in einem mit Wasser mischbaren organischen Lösungsmittel durchgeführt, welches auch mit Wasser vermischt sein kann. Das organische Lösungsmittel kann einzeln oder als Gemisch aus zwei oder mehreren Komponenten verwendet werden.

Beispiele von mit Wasser mischbaren organischen Lösungsmitteln sind aliphatische $C_1$–$C_3$-Alkohole wie Methanol, Ethanol oder die Propanole; Ketone wie Aceton oder Diacetonalkohol sowie auch N-Methylpyrrolidon.

Vorzugsweise arbeitet man in einem Lösungsmittelgemisch bestehend aus einem $C_1$–$C_3$-Alkanol und Wasser oder aus einem $C_1$–$C_3$-Alkanol und Aceton wie z.B. Ethanol/Wasser, Isopropanol/Wasser, Ethanol/Methanol/Wasser, Ethanol/Isopropanol/Wasser oder Ethanol/Aceton.

Zur Durchführung der Copolymerisation wird zweckmässigerweise zuerst eine Lösung der Monomerenmischung vorbereitet. Es können auch getrennte Lösungen der entsprechenden Monomeren eingesetzt werden.

Die Copolymerisation erfolgt in der Regel in Gegenwart eines Polymerisationsinitiators, welcher in den Lösungen der Monomeren vorhanden sein kann oder vorzugsweise in Form einer alkanolischen Lösung der Reaktionsmischung zugegeben wird.

Die Zusatzmengen, in denen der Polymerisationsinitiator der Reaktionsmischung zugesetzt wird, bewegen sich zwischen 0,001 und 0,5 Gew.-%, bezogen auf das Monomerengemisch. Geeignete Polymerisationsinitiatoren sind z.B. Azoisobutyronitril oder insbesondere organische Peroxide wie Dicumylperoxid, Di-tert.Butylper-

oxid, Diacetylperoxid, Dibenzoylperoxid, Benzoyl-acetylperoxid, Dilauroylperoxid, t.-Butylperben-zoat, tert.-Butylperoxy-neodecanoat oder vor allem tert.-Butylperoctoat.

Die Copolymerisation erfolgt im allgemeinen bei erhöhter Temperatur von z.B. 50 bis 100°C, insbesondere 70 bis 90°C unter Rückfluss und in inerter Atmosphäre, d.h. unter Verwendung eines inerten Gases, wie z.B. Stickstoff, das auch 2,5 bis 10 Vol-%, vorzugsweise 5 bis 6 Vol-% Sauerstoff enthalten kann.

Nach der Polymerisationsstufe wird die erhaltene Lösung des Terpolymeren auf Wasser zugegeben, worauf das Terpolymer in Form eines mehr oder weniger feinen Granulats ausgeschieden wird. Dies geschieht vorteilhafterweise bei einer Temperatur von 0 bis 40°C, vorzugsweise 10 bis 30°C. Die Isolierung des Polymerisationsproduktes erfolgt in allgemein bekannter Weise, z.B. durch Abfiltrieren des ausgeschiedenen Terpolymeren, Waschen und Trocknen. Die so erhaltenen Terpolymere haben ein Molekulargewicht von 15 000 bis 60 000, vorzugsweise 25 000 bis 35 000.

Diese Terpolymere können als Haarfixierungsmittel in Haarsprays verwendet werden. Daher sollte beachtet werden, dass die in den Terpolymeren vorhandenen freien Carboxylgruppen mindestens die Hälfte (im allgemeinen 55 bis 100%, vorzugsweise 75 bis 85%) neutralisiert in den fertigen Haarsprays vorliegen, damit die filmbildenden Polymerharze aus dem Haar durch einfaches Spülen oder durch Waschen entfernt werden können.

Zweckmässig erfolgt die Neutralisierung der Carboxylgruppen in der Weise, dass man die Terpolymere in Form einer Lösung in einem organischen Lösungsmittel, (z.B. $C_2$–$C_3$-Alkanole), das in der Kosmetik verwendet wird, mit oder ohne Zusatz von Wasser mit einer definitionsgemässen organischen Base umsetzt.

Bevorzugte organische Lösungsmittel sind Ethanol und Propanole. Diese können allein oder in Kombination mit 0,1 bis 20 Gew.-% Wasser oder 1 bis 70 Gew.-% anderer Lösungsmittel, z.B. Methylenchlorid oder Trichlorethan, verwendet werden. Weitere Cosolventien, die erfindungsgemäss eingesetzt werden können sind Alkylenglykolmononiederalkylether, wie z.B. Ethylenglykolmonomethylether, Ethylenglykolmonoethylether oder 1-Methoxypropanol sowie auch 2-Butanol oder 2,2-Dimethyl-4-hydroxymethyl-1,3-dioxalan. Bevorzugte Lösungsmittelkombinationen bestehen aus Ethanol und Methylenchlorid oder Isopropanol und Methylenchlorid, wobei gewünschtenfalls auch die genannten Cosolventien mitverwendet werden können.

Geeignete organische Basen sind Triethanolamin, insbesondere Triisopropanolamin und vor allem 2-Amino-2-methylpropanol oder 2-Amino-2-methyl-1,3-propandiol.

Zur Fertigstellung der erfindungsgemässen Haarfixierungszubereitung, die in Form eines Aerosol-Sprays verwendet wird, geht man so vor, dass man die nach der Behandlung des Terpolymeren mit der genannten organischen Base resultierende Lösung mit einem Aerosol-Treibmittel begast. Definitionsgemäss werden Treibmittel eingesetzt, welche mindestens 30 Gew.-%, vorzugsweise mindestens 50 Gew.-%, der gesamten Menge an Treibmittel aus einem halogenfreien Treibgas, insbesondere einem Kohlenwasserstofftreibmittel bestehen. Als weiteres halogenfreies Treibgas kann vorteilhafterweise Dimethylether verwendet werden.

Geeignete Kohlenwasserstofftreibmittel, welche in der erfindungsgemässen Zubereitung eingesetzt werden können, sind vorzugsweise Alkane, wie z.B. Propan, n-Butan, Isobutan, und Mischungen dieser Treibmittel, insbesondere eine Mischung von Propan und Butanen. Die Kohlenwasserstofftreibmittel können mit weiteren Treibgasen kombiniert werden, beispielsweise mit weiteren halogenfreien Treibgasen wie z.B. Dimethylether oder auch mit halogenhaltigen Treibmitteln wie 1,2-Dichlor-1,1,2,2-tetrafluorethan, Dichlordifluormethan oder vorzugsweise Trichlorfluormethan.

Bevorzugte erfindungsgemäss verwendbare Treibmittel sind halogenfreie Kohlenwasserstoffe mit 1 bis 5 Kohlenstoffatomen und insbesondere eine Mischung aus Propan und Butanen, vorzugsweise im Verhältnis von 1:10 bis 1:1,8. Propan und Butan können vorteilhafterweise in Kombination mit Dimethylether oder Trichlorfluormethan verwendet werden.

Die erfindungsgemässen fertigen Haarspray-Zubereitungen enthalten insbesondere, bezogen auf die gesamte Zubereitung, 0,5 bis 3 Gew.-% des neutralisierten Terpolymeren.

Der Anteil an Ethanol oder Isopropanol kann weniger als 20 Gew.-% der Formulierung, im Fall von Ethanol sogar auch nur 3 Gew.-% betragen.

Zur Verleihung von bestimmten Eigenschaften können gewünschtenfalls in die Haarfixierungszubereitungen Additive eingearbeitet werden. Beispiele dieser Additive sind Weichmacher, wie z.B. Phthalsäureester, Glykole und Glycerin, Gleitmittel und Durchdringungsmittel wie Lanoline, Proteinhydrolysate, und andere Proteinderivate, ferner Silikone, U.V.-Absorptionsmittel, Ethylenoxidaddukte, Polyoxyethylencholesterin sowie auch Geruchsstoffe und Farbstoffe. Insgesamt sollen diese Additive höchstens 20 Gew.-% des in der Zubereitung vorhandenen Terpolymer betragen.

Die Haarfixierungszubereitungen zeigen sämtliche kennzeichnenden Eigenschaften, welche für solche Produkte erforderlich sind. Die Filme sind transparent, glänzend, flexibel und fest. Sie besitzen gute antistatische Eigenschaften, haften gut an dem Haar und werden durch Tensidlösungen wie Shampoo leicht entfernt. Sie gestatten es, dass das Haar leicht gekämmt werden kann. Ausserdem vergilben sie beim Altern nicht, noch werden sie klebrig, wenn sie hohen Feuchtigkeiten ausgesetzt werden. Vor allem haben sie eine ausgezeichnete Lockenerhaltung bei hohen Feuchtigkeitsbedingungen.

In den folgenden Herstellungsvorschriften und Beispielen beziehen sich die angegebenen Prozentsätze, wenn nichts anderes angegeben ist, auf das Gewicht.

Herstellungsvorschriften

Vorschrift A

Eine Lösung (1), welche 75 Teile Acrylsäure, 420 Teile Acrylsäureethylester, 525 Teile N-tert.-Butylacrylamid in einem Gemisch aus 900 Teilen Ethanol und 400 Teilen Wasser enthält, wird vorbereitet und unter einer 6% Sauerstoff aufweisenden Stickstoffatmosphäre gelagert. Alsdann werden 10% der Lösung (1) in ein Reaktionsgefäss eingetragen, welches 100 Teile Ethanol und 40 Teile Wasser enthält, die auf Rückflusstemperatur aufgeheizt worden sind. Im Verlaufe von 1½ bis 2½ Stunden gibt man gleichzeitig die restlichen 90% der Lösung (1) und eine Lösung (2), die 8,9 Teile tert.-Butylperoctoat in 77 Teilen Ethanol enthält, zu. Die Beheizung des Reaktionsgefässes wird abgestellt und die Monomer- und Peroctoatzugabe so dosiert, dass durch die freiwerdende Reaktionswärme die Rückflusstemperatur aufrechterhalten wird. Dabei wird so vorgegangen, dass der Zulauf der Lösung (1) 15 bis 30 Minuten vor der Zugabe der Lösung (2) beendet ist. Nach Beendigung der Zugabe der Lösung (2) hält man die Temperatur noch 3 Stunden bei 80°C und lässt die Mischung bei dieser Temperatur ausreagieren. Anschliessend kühlt man auf 20–25°C und trägt die Reaktionsmasse unter Rühren in 7200 Teile Wasser ein. Man erhält einen körnigen, nicht klebenden Niederschlag, welcher abfiltriert, mit 5000 Teilen Wasser gewaschen und im Vakuum bei 40 bis 65°C, vorzugsweise 40 bis 50°C getrocknet wird.

Vorschriften B bis F

Weitere Polymere gemäss Vorschriften B bis F werden erhalten, wenn man nach dem in Vorschrift A beschriebenen Verfahren arbeitet und die in der folgenden Tabelle 1 aufgeführten Monomeren und Lösungsmittel in der angegebenen Menge (Gewichtsteilen) verwendet.

Tabelle 1

| Herstellungsvorschrift | B | C | D | E | F |
|---|---|---|---|---|---|
| N-tert.-Butylacrylamid | 578 | 350 | 350 | 535 | 350 |
| Acrylsäuremethylester | – | 242 | – | – | – |
| Acrylsäureethylester | 463 | – | – | – | 280 |
| Acrylsäurebutylester | – | – | 358 | 428 | – |
| Acrylsäure | 57,6 | 50 | 50 | 108 | 50 |
| Ethanol | 950 | 820 | 950 | 700 | 900 |
| Isopropanol | – | – | – | 200 | – |
| Aceton | – | – | – | – | 300 |
| Wasser | 400 | 400 | 250 | 280 | – |

Vorschrift G

In einem Reaktionsgefäss werden 35,5 g N-tert.-Butylacrylamid und 0,24 g α,α'-Azo-bis-isobutyronitril vorgelegt, worauf die Apparatur 15 Minuten mit Stickstoff gespült wird. Hierauf wird eine Lösung von 35,8 g Acrylsäurebutylester, 5,1 g Acrylsäure in 88 g Ethanol und 25 g Wasser in das Reaktionsgefäss gegeben. Man erwärmt auf 80°C, wobei das Gemisch an Comonomeren vollständig in Lösung geht und die Polymerisation einsetzt. Man rührt die Reaktionsmischung 5 Stunden bei 80°C, kühlt dann auf 20–25°C ab und fällt das Produkt durch Eintragen der Reaktionslösung in 750 g entionisiertes Wasser unter starker Umwälzung des Fällbades aus. Man erhält ein farbloses grobes Pulver, das abfiltriert und bei 60°C (im Vakuum) getrocknet wird.

Vorschrift H

In einem Reaktionsgefäss werden 30,6 g N-n-Butylacrylamid und 0,25 g α,α'-Azo-bis-isobutyronitril vorgelegt, worauf die Apparatur 15 Minuten mit Stickstoff gespült wird. Alsdann gibt man eine Lösung von 30,7 g n-Butylacrylester und 10,8 g Acrylsäure in 125 g Ethanol in das Reaktionsgefäss zu. Beim Erwärmen auf 80°C geht das Comonomeren-Gemisch vollständig in Lösung und die Polymerisation setzt ein. Das Reaktionsgemisch wird 5 Stunden bei 80°C gerührt, dann auf 20–25°C abgekühlt und in 2,5 Liter entionisiertes Wasser eingetragen, wodurch das Terpolymer als farbloses, grobes Pulver ausfällt. Dieses wird abfiltriert, mit 1,5 Liter Wasser gewaschen und bei 45–50°C im Vakuum getrocknet.

Vorschriften I bis O

Weitere Polymere gemäss Vorschriften I bis O werden erhalten, wenn man wie in Vorschrift H verfährt und die in der folgenden Tabelle 2 aufgeführten Monomeren in der angegebenen Menge (Gewichtsteile) verwendet.

Beispiel 1:

Eine Lösung von 1,5 Teilen des gemäss Vorschrift A hergestellten Terpolymeren (Polymer A) in 38,38 Teilen wasserfreiem Ethanol wird mit 0,12 Teilen 2-Amino-2-methylpropanol versetzt, wobei ca. 80% der im Terpolymeren vorhandenen Carboxylgruppen neutralisiert werden. Danach wird die Lösung in einen Aerosol-Behälter eingebracht, in welchen dann 60 Teile Propan/Butan (15:85) gedrückt werden. Man erhält eine klare Aerosolformulierung.

Tabelle 2

| Herstellungsvorschrift | I | K | L | M | N | O |
|---|---|---|---|---|---|---|
| N-tert.-Butylacrylamid | – | – | 30,6 | 35,1 | 35,5 | – |
| N-n-Butylmethacrylamid | – | – | – | – | – | 43,8 |
| N-Iso-propylacrylamid | 36,3 | – | – | – | – | – |
| N-Methylmethacrylamid | – | 25,0 | – | – | – | – |
| Acrylsäureethylester | 28,4 | – | 30,7 | 27,7 | – | 28,4 |
| Acrylsäureisobutylester | – | 40,4 | – | – | – | – |
| Methacrylsäureisobutyl-ester | – | – | – | – | 40,4 | – |
| Acrylsäure | 5,1 | – | 8,6 | 7,0 | – | – |
| Methacrylsäure | – | 6,1 | – | – | 6,1 | 6,1 |

Beispiele 2 bis 16:

Weitere klare Haarfixierungszubereitungen gemäss Beispielen 2 bis 16 werden erhalten, wenn man nach dem im Beispiel 1 beschriebenen Verfahren arbeitet und die in den folgenden Tabellen 3 und 4 aufgeführten Polymere, Basen, Lösungsmittel und Treibgase in der angegebenen Menge (Gewichtsteile) verwendet.

Tabelle 3

| Beispiel Nr. | 2 | 3 | 4 | 5 | 6 | 7 | 8 | 9 |
|---|---|---|---|---|---|---|---|---|
| Polymer A | 1,5 | – | – | 2,0 | 2,0 | 1,5 | 2,0 | 2,0 |
| Polymer C | – | 2,0 | – | – | – | – | – | – |
| Polymer E | – | – | 1,30 | – | – | – | – | – |
| Ethanol 96 Vol.% | – | 30,0 | – | – | – | – | – | – |
| Ethanol absolut | – | – | 33,57 | – | – | 23,38 | – | – |
| Isopropanol | 13,38 | – | – | 5,0 | 10,0 | – | 12,8 | 17,9 |
| Methylenchlorid | 20,0 | – | – | 35,0 | 35,0 | – | 35,0 | 30,0 |
| Wasser | – | 7,78 | – | – | – | – | – | – |
| Aminomethylpropanol | – | – | 0,13 | 0,15 | 0,15 | 0,12 | 0,2 | 0,1 |
| Aminomethylpropandiol | 0,12 | 0,22 | – | – | – | – | – | – |
| Propan/Butan (15 : 85) | 30,0 | – | 25,0 | 37,85 | 32,85 | 30,0 | 50,0 | 50,0 |
| Trichlorfluormethan | 35,0 | – | 40,0 | 20,0 | 20,0 | 45,0 | – | – |
| Dimethylether | – | 60,0 | – | – | – | – | – | – |

Tabelle 4

| Beispiel | 10 | 11 | 12 | 13 | 14 | 15 | 16 |
|---|---|---|---|---|---|---|---|
| Polymer A | 2,0 | 1,5 | 2,0 | 2,0 | 2,0 | 2,0 | 2,0 |
| Aminomethylpropanol | 0,2 | 0,12 | 0,15 | 0,15 | 0,15 | 0,15 | 0,15 |
| Ethanol absolut | – | – | 3,0 | 3,0 | 3,0 | 3,0 | 3,0 |
| Isopropanol | 32,8 | 13,38 | – | – | – | – | – |
| Methylenchlorid | 15,0 | 35,0 | 35,0 | 35,0 | 35,0 | 35,0 | 35,0 |
| Trichlorfluormethan | – | 20,0 | 20,0 | 20,0 | 20,0 | 20,0 | 20,0 |
| Propan/Butan (15 : 85) | 50,0 | – | 37,85 | 37,85 | 37,85 | 37,85 | 37,85 |
| Dimethylether | – | 30,0 | – | – | – | – | – |
| Butanol-2 | – | – | – | – | – | 2,0 | – |
| Ethylenglykolmono-methylether | – | – | 2,0 | – | – | – | – |
| Ethylenglykolmono-ethylether | – | – | – | 2,0 | – | – | – |
| *Solketal | – | – | – | – | – | – | 2,0 |
| 1-Methoxypropanol-2 | – | – | – | – | 2,0 | – | – |

* = 2-Dimethyl-4-hydroxymethyl-1,3-dioxalan.

Verträglichkeit der Polymeren mit Treibgasen.

Je eine isopropanolische und eine ethanolische Formulierung, welche jeweils 1,5% des Polymeren A und 0,12% 2-Amino-2-methylpropanol enthalten, werden gemäss den prozentualen Angaben der folgenden Tabelle 5 mit den Treibgasen Trichlor-fluormethan, Propan/Butan oder Dimethylether oder deren Kombination mit oder ohne Zusatz von Methylenchlorid oder Wasser in einem Aerosol-Behälter vermischt.

Tabelle 5

| Versuch Nr. | 1 | 2 | 3 | 4 | 5 |
|---|---|---|---|---|---|
| Polymer A | 1,5 | 1,5 | 1,5 | 1,5 | 1,5 |
| Alkanol* | 33,5 | 13,5 | 38,5 | 38,5 | 32,5 |
| Methylenchlorid | 35,0 | 20,0 | – | – | – |
| Wasser | – | – | – | – | 6,0 |
| Propan/Butan (15 : 85) | 30,0 | 30,0 | 20,0 | 60,0 | – |
| Trichlorfluormethan | – | 35,0 | 40,0 | – | – |
| Dimethylether | – | – | – | – | 60,0 |

* Alkanol kann Ethanol oder Isopropanol sein.

Löslichkeitsstabilität

Je ein gefüllter Behälter wurde bei Raumtemperatur und bei Wechseltemperatur (−20/+50°C) bei 12 Stunden-Intervall während 14 Tagen gelagert und anschliessend das Aussehen der Lösung bei −20°C, +20°C und +50°C visuell beurteilt.

Keine der zu Beginn der Prüfung klaren Lösungen zeigte bei der Beurteilung eine Veränderung.

Trübungspunkt

Die gefüllten Behälter wurden langsam bis auf −30°C abgekühlt. Keine der Testformulierungen zeigte bei dieser Temperatur eine Trübung (Beginn der Polymerfällung) (Standard bei −20 bis −25°C).

Zulässiger Treibmittelüberschuss

Nach einer Überdosierung des entsprechenden Treibgases um 50% zeigte keine der Testformulierungen eine Trübung, womit der Grenzwert für eine genügende Sicherheit vorhanden ist.

Die gemäss den Beispielen 1 bis 16 hergestellten Haarfixierungszubereitungen zeigen bei den obigen Versuchsbedingungen eine hervorragende Lösungsstabilität, die ein einwandfreies Funktionieren der Sprühdosen auch unter extremen klimatischen Bedingungen gewährleistet. Die Formulierungen geben dem Haar eine natürliche, elastische und dauerhafte Festigung, auch im feuchten Klima. Der Griff der Frisur bleibt auch nach wiederholter Anwendung weich und angenehm.

**Patentansprüche**

1. Haarfixierungszubereitung dadurch gekennzeichnet, dass sie
(1) eine Lösung von mindestens einem Terpolymeren in einem wasserfreien oder wasserhaltigen organischen Lösungsmittel, wobei das Terpolymer durch Copolymerisation
(a) von 40 bis 60 Gew.-% eines im Alkylteil 1 bis 4 Kohlenstofatome aufweisenden N-Alkylacrylamides oder N-Alkylmethacrylamides mit

(b) 35 bis 50 Gew.-% eines $C_1$–$C_4$-Alkylesters oder $C_1$–$C_4$-Hydroxylalkylesters der Acrylsäure oder Methacrylsäure und
(c) 3 bis 11 Gew.-% Acrylsäure oder Methacrylsäure, bezogen auf das Gesamtgewicht des Monomerengemisches, hergestellt und ein Anteil von mindestens 50% der im Terpolymer vorhandenen Carboxylgruppen mit einer niederen organischen Base ausgewählt aus 2-Amino-2-methyl-propanol, 2-Amino-2-methyl-1,3-propandiol, Triethanolamin und Triisopropanolamin neutralisiert worden ist, und
(2) ein Treibmittel, welches aus mindestens 30 Gew.-%, bezogen auf das gesamte Treibmittel, eines halogenfreien Treibgases besteht, enthält.

2. Zubereitung gemäss Anspruch 1, dadurch gekennzeichnet, dass die Komponente (a) N-tert.-Butylacrylamid ist.

3. Zubereitung gemäss einem der Ansprüche 1 und 2, dadurch gekennzeichnet, dass die Komponente (b) Acrylsäureethylester ist.

4. Zubereitung gemäss einem der Ansprüche 1 bis 3, dadurch gekennzeichnet, dass die Komponente (c) Acrylsäure ist.

5. Zubereitung gemäss einem der Ansprüche 1 bis 4, dadurch gekennzeichnet, dass als organisches Lösungsmittel Ethanol oder Isopropanol oder ein Gemisch davon verwendet wird.

6. Zubereitung gemäss einem der Ansprüche 1 bis 5, dadurch gekennzeichnet, dass als organische Base 2-Amino-2-methylpropanol oder 2-Amino-2-methyl-1,3-propandiol verwendet wird.

7. Zubereitung gemäss einem der Ansprüche 1 bis 6, dadurch gekennzeichnet, dass 55 bis 100%, vorzugsweise 75 bis 85% der Carboxylgruppen neutralisiert worden sind.

8. Zubereitung gemäss einem der Ansprüche 1 bis 7, dadurch gekennzeichnet, dass als Treibmittel ein halogenfreier Kohlenwasserstoff oder Dimethylether verwendet wird.

9. Zubereitung gemäss Anspruch 8, dadurch gekennzeichnet, dass als Treibmittel Propan, Butan oder Isobutan oder eine Mischung davon verwendet wird.

10. Verfahren zur Herstellung einer Haarfixierungszubereitung gemäss Anspruch 1, dadurch gekennzeichnet, dass man mindestens ein Terpolymer, wie es in Anspruch 1 definiert ist, in einem wasserfreien oder wasserhaltigen organischen Lösungsmittel löst oder dispergiert, das Terpolymer mit einer niederen organischen Base ausgewählt aus 2-Amino-2-methylpropanol, 2-Amino-2-methyl-1,3-propandiol, Triethanolamin und Triisopropanolamin in mindestens 50% der vorhandenen Carboxylgruppen neutralisiert und die resultierende Lösung des neutralisierten Terpolymeren nach allfälliger Zugabe von weiteren Additiven mit einem Treibmittel, welches aus mindestens 30 Gew.-% eines halogenfreien Treibgases, vorzugsweise eines Kohlenwasserstoffes, bezogen auf das gesamte Treibmittel, besteht, vermischt.

**Claims**

1. A hair-setting preparation which contains

(1) a solution of at least one terpolymer in an anhydrous or water-containing organic solvent, the terpolymer having been prepared by the copolymerisation of

(a) 40 to 60% by weight of an N-alkylacrylamide or N-alkylmethacrylamide having 1 to 4 carbon atoms in the alkyl moiety, with

(b) 35 to 50% by weight of a $C_1$–$C_4$alkyl ester or $C_1$–$C_4$hydroxyalkyl ester of acrylic acid or methacrylic acid, and with

(c) 3 to 11% by weight of an acrylic acid or methacrylic acid, based on the total weight of the mixture of monomers, with at least 50% of the carboxyl groups in the terpolymer having been neutralised by a lower organic base selected from 2-amino-2-methylpropanol, 2-amino-2-methyl-1,3-propanediol, triethanolamine and triisopropanolamine, and

(2) a propellent which consists of at least 30% by weight, based on the total propellent, of a halogen-free propellent gas.

2. A preparation according to claim 1 wherein component (a) is N-tert-butylacrylamide.

3. A preparation according to either of claims 1 or 2, wherein component (b) is ethyl acrylate.

4. A preparation according to any one of claims 1 to 3, wherein component (c) is acrylic acid.

5. A preparation according to any one of claims 1 to 4, wherein the organic solvent is ethanol or isopropanol, or a mixture thereof.

6. A preparation according to any one of claims 1 to 5, wherein the organic base is 2-amino-2-methylpropanol or 2-amino-2-methyl-1,3-propanediol.

7. A preparation according to any one of claims 1 to 6, wherein 55 to 100%, preferably 75 to 85%, of the carboxyl groups have been neutralised.

8. A preparation according to any one of claims 1 to 7, wherein the propellent is a halogen-free hydrocarbon or dimethyl ether.

9. A preparation according to claim 8, wherein the propellent is propane, butane or isobutane, or a mixture thereof.

10. A process for the preparation of a hair-setting preparation according to claim 1, which comprises dissolving or dispersing at least one terpolymer, as defined in claim 1, in an anhydrous or water-containing organic solvent, neutralising at least 50% of the carboxyl groups of the terpolymer with a lower organic base selected from 2-amino-2-methylpropanol, 2-amino-2-methyl-1,3-propanediol, triethanolamine and triisopropanol amine, and mixing the resultant solution of the neutralised terpolymer, after the optional addition of further additives, with a propellent which consists of at least 30% by weight of a halogen-free propellent gas, preferably a hydrocarbon, based on the total propellent.

**Revendications**

1. Préparation pour fixage des cheveux, caractérisée en ce qu'elle contient

(1) une solution d'au moins un terpolymère dans un solvant organique anhydre ou contenant de l'eau, le terpolymère ayant été préparé par copolymérisation

(a) de 40 à 60% en poids d'un N-alkylacrylamide ou d'un N-alkylméthacrylamide présentant 1 à 4 atomes de carbone dans la partie alkyle, avec

(b) 35 à 50% en poids d'un ester d'alkyle en $C_1$–$C_4$ ou d'un ester d'hydroxylalkyle en $C_1$–$C_4$ de l'acide acrylique ou de l'acide méthacrylique et

(c) de 3 à 11% en poids d'acide acrylique ou d'acide méthacrylique, par rapport au poids total du mélange de monomères, et une proportion d'au moins 50% en poids des groupes carboxyles présents dans le terpolymère ayant été neutralisés par une base organique inférieure choisie parmi le 2-amino-2-méthyl-propanol, le 2-amino-2-méthyl-1,3-propanediol, la triéthanolamine et la triisopropanolamine, et

(2) un propulseur, qui se compose d'au moins 30% en poids, par rapport au propulseur total, d'un gaz propulseur exempt d'halogène.

2. Préparation selon la revendication 1, caractérisée en ce que le constituant (a) est le N-tert-butylacrylamide.

3. Préparation selon l'une des revendications 1 ou 2, caractérisée en ce que le constituant (b) est l'ester éthylique de l'acide acrylique.

4. Préparation selon l'une des revendications 1 à 3, caractérisée en ce que le constituant (c) est l'acide acrylique.

5. Préparation selon l'une des revendications 1 à 4, caractérisée en ce que l'on utilise comme solvant organique l'éthanol ou l'isopropanol ou un mélange de ceux-ci.

6. Préparation selon l'une des revendications 1 à 5, caractérisée en ce qu'on utilise comme base organique le 2-amino-2-méthylpropanol ou le 2-amino-2-méthyl-1,3-propanediol.

7. Préparation selon l'une des revendications 1 à 6, caractérisée en ce qu'on a neutralisé 55 à 100%, de préférence 75 à 85%, des groupes carboxyles.

8. Préparation selon l'une des revendications 1 à 7, caractérisée en ce qu'on utilise comme propulseur un hydrocarbure exempt d'halogène ou l'éther de diméthyle.

9. Préparation selon la revendication 8, caractérisée en ce qu'on utilise comme propulseur du propane, du butane ou de l'isobutane ou un mélange de ceux-ci.

10. Procédé de préparation d'une composition pour fixage des cheveux selon la revendication 1, caractérisé en ce qu'on dissout ou disperse au moins un terpolymère, tel que défini dans la revendication 1, dans un solvant organique anhydre ou contenant de l'eau, en ce qu'on neutralise le terpolymère avec une base organique inférieure choisie parmi le 2-amino-2-méthylpropanol, le 2-amino-2-méthyl-1,3-propanediol, la triéthanolamine et la triisopropanolamine sur au moins 50% des groupes carboxyles présents, et en ce qu'on mélange la solution du terpolymère neutralisé obtenue, après addition éventuelle d'autres additifs, avec un propulseur qui se compose d'au moins 30% en poids d'un gaz propulseur exempt d'halogène, de préférence un hydrocarbure, par rapport au propulseur total.